# EUROPEAN PATENT SPECIFICATION

(11) **EP 1 232 748 B1**
(45) Date of publication and mention of the grant of the patent: **03.05.2006**
(21) Application number: 00972557.3
(22) Date of filing: 03.11.2000
(51) Int. Cl.: A61K 31/04, A61P 1/00, A61P 5/50, A61P 7/00, A61P 9/00, A61P 11/00, A61P 15/00, A61P 17/00, A61P 19/00, A61P 21/00, A61P 25/00, A61P 31/00, A61P 33/00, A61P 35/00, A61P 37/00

(54) **USE OF SUBSTITUTED NITROBENZENE DERIVATIVES FOR THE TREATMENT OF DISEASES CAUSED BY BACTERIA, FUNGI AND VIRUSES**
VERWENDUNG VON SUBSTITUIERTEN NITROBENZOL DERIVATEN ZUR BEHANDLUNG VON ERKRANKUNGEN VERURSACHT VON BAKTERIEN, PILZEN UND VIREN
UTILISATION DE DERIVES DE NITROBENZENE DANS LE TRAITEMENT DES MALADIES CAUSEES PAR LES BACTERIES, LES CHAMPIGNONS ET LES VIRUSES

(30) Priority: 05.11.1999 CN 99123684
(43) Date of publication of application: 21.08.2002
(73) Proprietor: Men, Tewa, Beijing 100054 (CN); Men, Yangyang, Beijing 100054 (CN)
(72) Inventor: Men, Tewa, Beijing 100054 (CN); Men, Yangyang, Beijing 100054 (CN)
(74) Representative: Popp, Eugen
(86) International application number: PCT/CN2000/000416
(87) International publication number: WO 2001/037823

(56) References cited:
- CN-A- 1 121 832
- US-A- 2 023 565
- RIEDEL J ET AL: "Biotransformation and toxicokinetics of musk xylene in humans." TOXICOLOGY AND APPLIED PHARMACOLOGY. UNITED STATES 1 JUN 1999, vol. 157, no. 2, 1 June 1999 (1999-06-01), pages 145-155, XP002275484 ISSN: 0041-008X
- ZHENG GUO QIANG ET AL.: 'Isolation and biological evaluation of potential cancer chemopreventive agents from ambrette musk residue' J. PHARM. SCI. vol. 81, no. 9, September 1992, pages 950 - 953, XP002955367

## Description

### FIELD OF THE INVENTION

The present invention relates to substituted nitrobenzene derivatives of general Formula I as medicine and health food, a pharmaceutical composition comprising a substituted nitrobenzene derivative of general Formula I and the use thereof for the prophylaxis and treatment of diseases.

### BACKGROUND ART

Nippon Kagaku Zaeshi 80, 476-86 (1953) discloses some nitrobenzene compounds, but nothing is reported about any use thereof. Wpi Derwent abstract of CN-A-1121832 and J. pharm. Sci, 1992, 81, 9, pages 950-953 respectively describe the use of 2,6-dimethyl-4-*tert*-butyl-nitrobenzene, 2,6-dimethyl-4-*tert*-butyl-1,3-dinitrobenzene, 2,6-dimethyl-4-tert pentylnitrobenzene, 2-methyl-6-ethyl-4-*tert-*butylnitrobenzene and 1-*tert*-butpl-3,5-dimethyl-2,4,6-trinitrobenzene, to treat cancer.

None of these documents discloses or suggests the use of the nitrobenzene derivatives of the present invention to treat diseases caused by viruses fungi and bacteria.

### OBJECTIVE OF THE INVENTION

The objective of the present invention is to develop a use of substituted nitrobenzene derivatives.

### SUMMARY OF THE INVENTION

The present invention as defined by claims 1 to 11 relate to a use of at least one compound of Formula I in the manufacture of pharmaceutical composition for the prophylaxis and treatment of diseases , injuries or symptoms caused by bacteria, fungi, viruses, 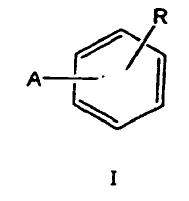 wherein, A is one, two or three nitro groups, at 1, 3, or 5 position on the benzene ring; R is one, two or three linear or branched C₁₋₆ alkyl groups, at 2, 4 or 6 position on the benzene ring.

### DETAILED DESCRIPTION OF THE INVENTION

The term "animals" used in the present description denotes mammal, especially human.

The term "linear or branched C₁₋₆ alkyl groups" used in the present description denotes linear or branched alkyl groups containing 1-6 carbon atoms, e.g. methyl, ethyl, propyl, isopropyl, *n*-butyl, isobutyl, *sec*-butyl, *tert*-butyl, amyl, neoamyl or hexyl.

The term "diseases or symptoms" used in the present description embraces diseases or symptoms caused by bacteria, fungi and viruses. Said diseases or symptoms include, virus hepatitis, tuberculosis, dysentery, poliomyelitis, chickenpox, herpes zoster, parotitis, cholera, influenza, scarlet fever, diphtheria, pertussis, typhoid, viral encephalitis, malaria, visceral leishmaniasis, brucellosis, rabies, plague, anthrax, leptospirosis, conjunctivitis, otitis media, paranasal sinusitis, stomatitis, pharyngitis, gingivitis, periodontitis, tonsillitis, parotitis, cholecystitis, cystitis, pancreatitis, arteritis, phlebitis, vaginitis, vulvitis, orchitis, salpingitis, ovaritis, endometritis, tumor of cervix, prostatitis, soft tissue inflammation, osteomyelitis, myelitis, peritonitis, cecitis, psoriasis, dermatasclerosis, viral dermatosis, coccigenic dermatosis, bacillary dermatosis, fungal dermatosis, spirochetal dermatosis, zoogenetic dermatosis, occupational dermatosis, neurotic dermatosis, allergodermia, physicogenic dermatosis, photogenous dermatosis, allergic dermatosis, lichen planus and lichenoid rash, erythemoid dermatosis, erythroderma desquamativum, vesicular dermatosis, keratinizing dermatosis, pigmentary dermatosis, dermovascular disease and lymphodermia, nutritional and metabolic dermatosis, disease of dermis and hypodermis, reticulosis, skin syndrom, granuloma annulare, pustuloderma, dermatitis continuee of extremities, edeomycodermatitis such as cervical erosion, neurodermatitis and ulcerative disease of gastrointestinal trast and mouth cavity.

The compounds of Formula I according to the present invention, wherein A is one nitro group at 1 position on the benzene ring, R is CH₃, C(CH₃)₃ and CH₃ at 2, 4 and 6 position respectively on the benzene ring, are preferred.

The compounds of Formula I according to the present invention, wherein A is preferably three nitro groups at 1, 3 and 5 position on the benzene ring, R is preferably CH₃, C(CH₃)₃ and CH₃ at 2, 4 and 6 position respectively on the benzene ring.

The compounds of Formula I according to the present invention, wherein A is preferably two nitro groups at 3 and 5 position on the benzene ring, R is preferably CH₃, C(CH₃)₃ and CH₃ at 2, 4 and 6 position respectively on the benzene ring.

The compounds of Formula I according to the present invention, wherein A is preferably one nitro group at 1 position on the benzene ring, R is preferably C(CH₃)₃, CH₃, and CH₃ at 2, 4 and 6 position respectively on the benzene ring.

The compounds of Formula I according to the present invention, are preferably selected from the following group:
1,3,5-trinitro-2,6-dimethyl-4-*tert*-butyl benzene;
1,3-dinitro-2,6-dimethyl-4-*tert*-butyl benzene;
1,5-dinitro-2,6-dimethyl-4-*tert*-butyl benzene;
3,5-dinitro-2,6-dimethyl-4-*tert*-butyl benzene;
1-nitro-2,6-dimethyl-4-*tert*-butyl benzene;
3-nitro-2,6-dimethyl-4-*tert*-butyl benzene;
5-nitro-2,6-dimethyl-4-*tert*-butyl benzene;
1,3,5-trinitro-2,4,6-*tert*-butyl benzene;
1,3-dinitro-2,4,6-*tri-tert*-butyl benzene;
3,5-dinitro-2,4,6-*tri-tert*-butyl benzene;
1,5-dinitro-2,4,6-*tri-tert*-butyl benzene;
1-nitro-2,4,6-tri-*tert*-butyl benzene;
3-nitro-2,4,6-*tri-tert*-butyl benzene;
5-nitro-2,4,6-*tri-tert*-butyl benzene;
1,3,5-trinitro-2,4,6-tri-methyl benzene;
1,3-dinitro-2,4,6-tri-methyl benzene;
3,5-dinitro-2,4,6-tri-methyl benzene;
1,5-dinitro-2,4,6-tri-methyl benzene;
1-nitro-2,4,6-tri-methyl benzene;
3-nitro-2,4,6-tri-methyl benzene;
5-nitro-2,4,6-tri-methyl benzene; and
1-nitro-2,6-dimethyl-4- *tert*-butyl benzene

According to the present invention, it is found that the compounds of Formula I may act selectively on the metabolic processes of important bio-active substances through the control of central nervous system-mediated body information network, to enhance the synthesis of physio-active substances which is responsible for the regulation of metabolism and bio-active substances which resist the endogenous and exogenous pathogenic factors, and to accelerate the cell division as well, resulting in active repair of injury and prompt improvement of the defense function against pathogenic factors, including barrier action, immune surveillance and recognition, phagocytosis and killing, detoxification and clearing, specific and non-specific immunity, while directly clearing the pathogenic factors. In detail, said compound of Formula I may exhibit beneficial bio-activities through the following ways:
1. Said compound of Formula I can rapidly improve specific and non-specific immunity, including humoral immunity, cellular immunity, phagocytosis of reticuloendothelial system and the activity of NK cell under both normal and abnormal conditions, and thus enhance the recognition, surveillance, killing, phagocytosis and clearance of such foreign bodies as bacteria, viruses and tumor cells. In particular, said compound of Formula I may, in sensitive animals, activate the cortex cells of thymus which belongs to central lymph organ and the precursor cells in bone marrow, promote cell division and proliferation of immunoblast, increase the number of lymphocyte in peripheral circulation, and promote the conversion of lymphocyte, the synthesis of antibodies by plasma cell, the phagocytosis of macrophage and phagocyte, while protect the stability of cell, avoid tissue lysis in the meantime, especially towards cytozoic pathogens such as viruses and *Mycobacterium tuberculosis*, tumor cells, and diseases caused by bacteria, fungi and viruses.
2. Said compound of Formula I can stabilize the lysosome membrane and decrease the release of hydrolase. Since hydrolase acts to decompose proteins, to cleave nucleic acid and mucopolysaccharide, leading to destruction of tissue cell, decomposition of connective tissue, and release of histamine, serotonin and kinin, which initiate a series of inflammatory reactions by angiectasis, increasing vascular permeability and aggregation of leucocytes, the stability of lysosome membrane may not only reduce the above mentioned inflammatory reactions, in particular, the exudation, edema and capillary hyperpermeability during the early phase of inflammation, tissue lysis by leucocytes and phagocytes, so as to alleviate such symptoms as redness, swelling, calor and tenderness, but also inhibit the hyperplasia of fibroblast during the late phase of inflammation to avoid granulation, adhesion and scarring. Also, it may improve or cure allergic diseased caused by endogenous histamine and produce antipruritic effect; inhibit the release of sensitizing substances by mastocyte to alleviate or eliminate the bronchospasm and myxedema, and thus produce antiasthmatic and antiussive effects. Due to the ability to improve stability of antibodies and lysosome membrane, said compound of Formula I may avoid and eliminate such allergic diseases as autoimmune diseases, anaphylaxis, and diseases due to connective tissue proliferation.
3. Said compounds of Formula I anticipate the tissue repair during the late phase of inflammation, in particular, enhance the regeneration and renew of tissue cells, such as smooth muscle cells, skeletal muscle cells, dermis and mucoca, inhibit fibroblast proliferation, to prevent the granulation and scarring, while leading to fast healing and delayed senility.
4. Said compounds of Formula I may act upon hemoglobin, platelet and granulocyte to enhance hematopoiesis e.g. synthesis of hemoglobin and cell division, and thus prevent and treat diseases of the blood system, alleviate fatigue, increase oxygen supply to heart, brain and other tissue cells, improve sleeping disorders, memory or intelligence.
5. Said compounds of Formula I may act selectively on the genetic center of enkephalin, through specific binding to the opium receptors, to achieve analgesia and abstinence.
6. Said compounds of Formula I may selectively participate the process of bio-synthesis and release of neurotransmitter, to coordinate the functions of cholinergic and adrenergic nerves, to regulate the functions of vegetative and motor nerves, e.g. visceral smooth muscle, gland effector, skeletal muscle, vessels and cardiac muscle, and thus help the body to rest and accumulate energy, to regulate the blood system, heart rate, digestive function, exocrine and endocrine of the body.
7. Said compounds of Formula I also participate the metabolism of bio-catalyst, i.e. various bio-enzymes e.g. synthase, decomposase, tool enzyme, steapsin, pancreatic amylase, trypsase, etc, and the secretion of insulin, and thus regulate the metabolism of lipid, carbohydrate and protein, to prevent and treat diseases or symptoms related to abnormal metabolism of lipid, carbohydrate and protein, e.g. diabetes mellitus, fatty liver, gout, atherosclerosis, etc, as well as detoxification in cases of ethylism, snakebite and bee sting.

Therefore, the present invention relates to compounds of Formula I preferably used for the prophylaxis and/or treatment of diseases, injury or symptoms cause by bacteria, fungi and viruses. wherein A and R are defined as above.

More particularly, according to the present invention, compounds of Formula I are used for the preparation of pharmaceuticals for the prophylaxis and treatment of virus hepatitis, tuberculosis, poliomyelitis, typhoid, bacillary dysentery, cholera, influenza, chickenpox, herpes zoster, parotitis, scarlet fever, diphtheria, pertussis, encephalitis B, viral encephalitis, malaria, visceral leishmaniasis, rabies, plague, anthrax, brucellosis, leptospirosis, osteomyelitis, encephalitis, optic neuritis, conjunctivitis, otitis media, paranasal sinusitis, stomatitis, pharyngitis, gingivitis, periodontitis, tonsillitis, bronchitis, pneumonia, gastroenteritis, cholecystitis, cystitis, pancreatitis, arteritis, phlebitis, neuritis, vaginitis, vulvitis, orchitis, salpingitis, ovaritis, endometritis, prostatitis, psoriasis, viral dermatosis, coccigenic dermatosis, bacillary dermatosis, fungal dermatosis, spirochetal dermatosis, zoogenetic dermatosis, occupational dermatosis, neurotic dermatosis, allergodermia, physicogenic dermatosis, photogenous dermatosis, allergic dermatosis, lichen planus and lichenoid rash, erythemoid dermatosis, erythroderma desquamativum, vesicular dermatosis, keratinizing dermatosis, pigmentary dermatosis, dermovascular disease and lymphodermia, nutritional and metabolic dermatosis, disease of dermis and hypodermis, reticulosis, skin syndrom, granuloma annulare, pustuloderma, dermatitis continuee of extremities, edeomycodermatitis such as cervical erosion, neurodermatitis and ulcerative disease of gastrointestinal tract and mouth cavity.

The present invention also relates to pharmaceutical compositions comprising at least one compound of Formula I as the active ingredient, pharmaceutical carrier or excipient. The present pharmaceutical compositions usually contain 0.1-90% by weight compounds of Formula I. According to the present invention, said pharmaceutical compositions may be prepared by known methods in the art. Said compounds of Formula I may be prepared in combination with one or several solid or liquid pharmaceutical carriers and/or excipients, in suitable administration route and dosage form for human and veterinary application as needed.

According to the present invention, said compounds of Formula I or the pharmaceutical compositions comprising them, may be administered, in unit dosage form, intestinally, parenterally or topically, e.g. orally, intramuscularly, subcutaneously, intranasally, by oral absorption, intravenously, percutaneously, intraperitoneally, rectally, etc. Administrative dosage are as follows: tablet, capsule, drop, aerosol, pill, powder, liquor, suspension, emulsion, granule, ointment, suppository, freeze-dried injection, etc, which could be regular preparation, delayed-released preparation, controlled-released preparation and various micro-granule delivery system. In case of tablet, various carriers known in the art may be used, e.g. dilutent and resorbent such as starch, dextrin, calcium sulfate, kaolin, microcrystalline cellulose, aluminium silicate, etc; wetting agent and adhesives such as water, glycerin, polyethylene glycol, ethanol, propanol, starch mucilage, dextrin, syrup, honey, glucose solution, acacia, gelatin, carboxymethylcellulose sodium, shellac, methylcellulose, potassium phosphate, polyvinylpyrrolidone, etc; disintegrating agent, such as dried starch, alginate, agar powder, laminaran, sodium bicarbonate and citric acid, calcium carbonate, polyoxyethylene sorbitol aliphatic ester, lauryl sodium sulfate, methylcellulose, ethylcellulose, etc; disintegration inhibiting agent, such as sucrose, tristearin, cacao butter, hydrogenated oil, etc; absorption accelerator, such as quaternary ammonium salt, lauryl sodium sulfate, etc; lubricant, such as talc, silica, corn starch, stearate, boric acid, fluid wax, polyethylene, etc. The tablet may be further formulated into coated tablet, e.g. sugar-coated tablet, film-coated tablet, enteric-coated tablet, or double-layer tablet and multi-layer tablet. In the case of pill, various carriers known in the art may be used, e.g. dilutent and resorbent, such as glucose, lactose, starch, cacao butter, hydrogenated vegetable oil, polyvinylpyrrolidone, kaolin, talc, etc; adhesives, such as acacia, bassora gum, gelatin, ethanol, honey, liquid sugar, rice paste or flour paste, etc; disintegrating agent, such as agar powder, dried starch, alginate, lauryl sodium sulfate, methylcellulose, ethylcellulose. In case of suppository, various carriers known in the art may be used, e.g. polyethylene, lecithin, cacao butter, higher alcohols, esters of higher alcohols, gelatin, semi-synthetic glyceride, etc. In the case of capsule, it may be prepared by mixing said compounds of Formula I as active ingredient with the above mentioned carriers, followed by placing the mixture into a hard gelatin capsule or soft capsule. Also, said compounds of Formula I may be applied in the following dosage forms: microcapsules, suspension in an aqueous phase, hard capsule, or injection. In the case of injection, such as liquor, emulsion, freeze-dried injection, and suspension, all the dilutents common in the art may be used, e.g. water, ethanol, polyethylene glycol, propylene glycol, oxyethylated isostearyl alcohol, polyoxidated isostearyl alcohol, polyoxyethylene sorbitol aliphatic ester, etc. In addition, in order to obtain isotonic injection, a suitable amount of sodium chloride, glucose or glycerin may be added into the preparation, as well as regular cosolvent, buffer, pH adjusting agent, etc.

In addition, coloring agent, antiseptic, perfume, correctives, food sweetening agent or other materials may be added to the pharmaceutical preparation if necessary.

According to the present invention, the dosage of said compounds of Formula I depends on a variety of factors, including the nature and severity of the diseases, the sex, age, weight and individual reaction of the subject, the particular compound employed, the route and frequency of administration, etc. Said compounds of Formula I or the pharmaceutical compositions comprising them may be administered in single or divided dosage form, e.g. one to four doses per day.

The following examples and biological activity assays or clinical trials are presented for further illustrative purposes only and are not intended as a restriction on the scope of the invention.

### I. Toxicologic study

### 1. Acute toxicity test

### (1) Oral administration

Mice, weight: 20g±2g, male and female half by half. Nitrobenzene compounds were prepared as an aqueous suspension with a concentration of 20% and given to the fast mice three times per day by gastrogavage. 0.3ml per time to a total of 18g/kg, corresponding 600 times of the daily dosage for human. No death or lethal adverse reaction was observed for successive 7days.

### (2) Injection administration

Mice, weight: 20g±2g, male and female half by half. Nitrobenzene compounds were prepared as an injection with a concentration of 2% and administered 0.4ml each. Results: LD₅₀(ip)=18.07±4.52g/kg, LD₅₀(iv)=14.19±211g/kg.

### 2. Long-term toxicity test

170 Wistar rats, weight: 86±8g, male and female half by half. Gastrogavage. Nitrobenzene compounds were prepared as an aqueous suspension. 0.6, 2.0, 6g/kg respectively (20, 70, 200 times the dosage for human). Continuous daily administration for 6 months. The mean gain in weight and the bioavailability rate of 3 experimental groups is higher, as compared with the control group. No significant difference was observed on peripheral hemogram, hepatic and renal function, and biochemical criteria such as blood sugar. No marked pathologic improvement was present in important organs. The results indicate that no lethal adverse reaction occur at the dosage of 6g/kg/d.

### 3. Genetic toxicity test, traditional teratogenicity test, 18-month-diet carcinogenic test. The results show that nitrobenzene compounds demonstrate no noticeable teratogenic, mutagenic and carcinogenic actions.

### II. Pharmacodynamics study

### Observations on the antiviral and antibacterial actions.

The results show that in case of 3 and 30TCTD₅₀ virus infection, said compounds at the dosage of 0.01g/kg demonstrate significant inhibition of cytopathy caused by influenza virus F_{M} strain, enterovirus E (HO₁₁) and herpes virus type I, and of virus replication in vivo in mice. The results of antibacterial test show that said compounds demonstrate significant inhibition of the following 8 bacteria tested: *Haemophilus influenzae, Streptococcus pneumoniae, Staphylococcus aureus, Escherichia coli, Pseudomonas aeruginosa, Mycobacterium tuberculosis, Shigella flexneri*, yeast.

### Synthetic methods of nitrobenzene compounds

1. *m*-xylene and *tert*-butyl alcohol in proportion of 1:3 were placed in the reactor. A suitable amount of aluminum trichloride was employ as catalyst. With cooling by circulating ice water, it was stirred and mixed well and react for over 2 hours. The reaction solution was poured slowly into concentrated hydrochloride, in proportion of 3:1. When the solution became clear and transparent, the reaction was completed. The resulting solution was subjected to extraction with chloroform/water, intensively mixed, stand, and the organic phase was added to sodium bicarbonate. The process was repeated until the pH reached 6-8. The residual was distilled to afford 2,6-dimethyl-4-*tert*-butyl benzene.
2. To 2,6-dimethyl-4*-tert*-butyl benzene solution in the reactor, an equal amount of acetic anhydride was added. After intensive stirring and mixing, nitric acid was added slowly under circulating cooling. The reaction sample was identified by T.L.C to determine the completeness of the reaction.
3. The reaction solution was extracted with chloroform. The organic phase was dried over sodium carbonate followed by filtration. Allow standing, white acicular crystals precipitated to afford the product 1-nitro-2,6-dimethyl-4-*tert*-butyl benzene. Molecular weight: 207.273, formula: C₁₂H₁₇NO₂.

All compounds are white or creamy crystals, soluble in organic solvent, but not in water, with a scent of volatile oil, mild in taste. Mp: 80-82°C. Other nitrobenzene compounds belong to known technology, and are no more repeated here.

### Example 1 suspension

100mg 1-nitro-2,6-dimethyl-4- *tert*-butyl benzene or 3,5-dinitro-2,6-dimethyl-4- *tert*-butyl benzene or 1,3,5-trinitro-2,6-dimethyl-4-*tert*-butyl benzene was accurately weighed and placed in a container and to it 100ml glycerin was added. The mixture was heated to achieve dissolution and intensively mixed. Distilled water was added up to 1000ml with intensive mixing to afford an emulsion with a concentration of 0.1%. It was then sealed and bottled, sterilized to give products. For external use or oral preparation.

### Example 2 emulsion

150mg 1-nitro-2,6-dimethyl-4- *tert*-butyl benzene or 3,5-dinitro-2,6-dimethyl-4- *tert*-butyl benzene or 1,3,5-trinitro-2,6-dimethyl-4-*tert*-butyl benzene was accurately weighed and placed in a container and to it 100ml sesame oil was added. The mixture was heated to achieve dissolution and intensively mixed. Distilled water was added up to 1000ml with intensive mixing to afford an emulsion with a concentration of 0.15%. It was then sealed and bottled, sterilized to give products. For external use or oral preparation.

### Example 3 aqueous for external use

200mg 1-nitro-2,6-dimethyl-4-*tert*-butyl benzene or 3,5-dinitro-2,6-dimethyl-4- *tert*-butyl benzene or 1,3,5-trinitro-2,6-dimethyl-4-*tert*-butyl benzene was accurately weighed and placed in a container and to it 100ml ethanol and a suitable amount of Tween were added. The mixture was heated to achieve dissolution and intensively mixed. Distilled water was added up to 1000ml with intensive mixing to afford an aqueous solution with a concentration of 0.2%. It was then sealed and bottled, sterilized to give products for external use.

### Example 4 capsule

300g 1-nitro-2,6-dimethyl-4-*tert*-butyl benzene or 3,5-dinitro-2,6-dimethyl-4- *tert*-butyl benzene or 1,3,5-trinitro-2,6-dimethyl-4-*tert-*butyl benzene, 300g black soya bean powder, 140g glucose, 60g albumin, 200g starch were weighed and subjected to grinding, mesh screening and intensive mixing, followed by capsule filling. Each capsule contained 0.2-0.33g content, for oral pharmaceuticals and health food.

### Example 5 injection

200mg 1-nitro-2,6-dimethyl-4-*tert*-butyl benzene or 3,5-dinitro-2,6-dimethyl-4-*tert*-butyl benzene or 1,3,5-trinitro-2,6-dimethyl-4-*tert-*butyl naphthalene(sodium salt) was accurately weighed and placed in a sterile container and to it 1000ml water for injection was added . The mixture was heated to achieve dissolution and packed in 2ml ampoules with a concentration of 4mg/2ml per ampoule. It was then sealed and sterilized to give products.

### Example 6 disinfectant

100mg 1-nitro-2,6-dimethyl-4-*tert*-butyl benzene or 3,5-dinitro-2,6-dimethyl-4- *tert*-butyl benzene or 1,3,5-trinitro-2,6-dimethyl-4-*tert*-butyl benzene was accurately weighed and placed in a container and to it 100ml a suitable amount of ethanol and Tween were added. The mixture was heated to achieve dissolution and intensively mixed. Distilled water was added up to 1000ml with intensive mixing to afford a disinfectant a concentration of 0.01%.

### Example 7 aqueous for external use

135mg 1-nitro-2,6-dimethyl-4-*tert*-butyl benzene or 3,5-dinitro-2,6-dimethyl-4-*tert*-butyl benzene or 1,3,5-trinitro-2,6-dimethyl-4-*tert*-butyl benzene was accurately weighed and placed in a container and to it ethanol was added up to 800ml followed by filtration. Still more ethanol was added through the filter to achieve 1000ml. The mixture was stirred well to give the preparation. It was then sealed and refrigerated to be stored.

### Example 8 shampoo

200mg 1-nitro-2,6-dimethyl-4-*tert*-butyl benzene or 3,5-dinitro-2,6-dimethyl-4-*tert*-butyl benzene or 1,3,5-trinitro-2,6-dimethyl-4-*tert*-butyl benzene was accurately weighed and placed in a container and to it 30g glycerin, 50ml ethanol were added and shaken well. Distilled water was added up to 2000ml with intensive mixing to afford shampoo with a concentration of 0.1%. It was applied in baldess, seborrheic dermatitis of scalp and for antipruritic use.

### Example 9 oral liquid

250mg 1-nitro-2,6-dimethyl-4-*tert*-butyl benzene or 3,5-dinitro-2,6-dimethyl-4-*tert*-butyl benzene or 1,3,5-trinitro-2,6-dimethyl-4-*tert*-butyl benzene was accurately weighed and placed in a container and to it 100ml sesame oil was added. After the mixture was heated to achieve dissolution, 200ml honey was added and stirred well. Distilled water was added up to 1000ml with intensive mixing to afford oral liquid with a concentration of 0.25%.

### Example 10 suppository

100mg 1-nitro-2,6-dimethyl-4-*tert*-butyl benzene or 3,5-dinitro-2,6-dimethyl-4-*tert*-butyl benzene or 1,3,5-trinitro-2,6-dimethyl-4-*tert*-butyl benzene was accurately weighed and to it melting semi-synthetic fatty glyceride was added fractionally, followed by quick stirring to the point of coagulation. It was then filled into suppository model, coagulated, scraped and then collected for packing.

### Example 11 skin emulsion

100mg 1-nitro-2,6-dimethyl-4-*tert*-butyl benzene or 3,5-dinitro-2,6-dimethyl-4-*tert*-butyl benzene or 1,3,5-trinitro-2,6-dimethyl-4-*tert*-butyl benzene was accurately weighed followed by grinding and mesh screening, and then placed in a mortar. Peanut oil was added and mixed well. Solution of calcium hydroxide was added slowly and ground to give a paste. More solution of calcium hydroxide was added up to 1000ml to afford a emulsion for external application.

### Example 12 ointment

100mg 1-nitro-2,6-dimethyl-4-*tert*-butyl benzene or 3,5-dinitro-2,6-dimethyl-4-*tert*-butyl benzene or 1,3,5-trinitro-2,6-dimethyl-4-*tert*-butyl benzene was accurately weighed followed by grinding and mesh screening, and then placed in a mortar. Glycerin was added and ground to achieve dissolution. Vaselin was added up to 1000g, and ground well to afford ointment for external application or skin protection.

### Example 13 powder

100mg 1-nitro-2,6-dimethyl-4-*tert*-butyl benzene or 3,5-dinitro-2,6-dimethyl-4-*tert*-butyl benzene or 1,3,5-trinitro-2,6-dimethyl-4-*tert-*butyl benzene was accurately weighed. Glucose was ground and mixed well followed by mesh screening to afford powder.

### Example 14 powder

100mg 1-nitro-2,6-dimethyl-4-*tert*-butyl benzene or 3,5-dinitro-2,6-dimethyl-4-*tert*-butyl benzene or 1,3,5-trinitro-2,6-dimethyl-4-*tert*-butyl benzene was accurately weighed and to it 200mg talc was added. Then boric acid and powder of zinc oxide, which were not ground in advance, were added in turn. A suitable amount of talc was added up to 200mg and mixed well to afford powder.

### Example 15 eye ointment

200mg 1-nitro-2,6-dimethyl-4-*tert*-butyl benzene or 3,5-dinitro-2,6-dimethyl-4-*tert*-butyl benzene or 1,3,5-trinitro-2,6-dimethyl-4-*tert*-butyl benzene was accurately weighed and placed in a sterile mortar. A small amount of sterile liquid wax was added and ground to give fine paste. Then the ground substance was added fractionally, accompanied by grinding, to achieve 1000g. It was then packed with aseptic manipulation, to afford eye ointment.

### Example 16 eye drops

50mg 1-nitro-2,6-dimethyl-4-*tert*-butyl benzene or 3,5-dinitro-2,6-dimethyl-4-*tert*-butyl benzene or 1,3,5-trinitro-2,6-dimethyl-4-*tert*-butyl benzene sodium was accurately weighed and placed in a sterile container, to which 100ml water for injection was added. The mixture was heated to achieve dissolution. Water for injection was added up to 500ml. It was then bottled, flow-steam sterilized for 30 minutes to afford eye drops.

### Example 17 ear drops

200mg 1-nitro-2,6-dimethyl-4-*tert*-butyl benzene or 3,5-dinitro-2,6-dimethyl-4-*tert*-butyl benzene or 1,3,5-trinitro-2,6-dimethyl-4-*tert*-butyl benzene was accurately weighed and to it 400ml ethanol was added. The mixture was heated to achieve dissolution followed by filtration. 300ml glycerin was added and stirred well. Distilled water was added up to 500ml. It was then packed to afford ear drops.

### Example 18 nose drops

200mg 1-nitro-2,6-dimethyl-4-*tert*-butyl benzene or 3,5-dinitro-2,6-dimethyl-4-*tert*-butyl benzene or 1,3,5-trinitro-2,6-dimethyl-4-*tert*-butyl benzene was accurately weighed and to it 40ml ethanol was added. The mixture was heated to achieve dissolution. A suitable amount of Tween 80 was added and stirred well. Distilled water was added up to 5000ml and mixed well to afford nose drops.

### Example 19 tablet

Prepared according to the method known in the art. 1-nitro-2,6-dimethyl-4-*tert*-butyl benzene or 3,5-dinitro-2,6-dimethyl-4-*tert*-butyl benzene or 1,3,5-trinitro-2,6-dimethyl-4-*tert*-butyl benzene and tablet, wherein said tablet comprised 1 %-2% said compounds according to effective demand. The content of said compounds may be increased or decreased as well. According to known technology and equipment, 300g compounds identified in the above examples, 200g microcrystalline cellulose, 300g anhydrous lactose, 200g magnesium stearate, were formulated to afford tablet.

### Example 20 oral liquid

500mg 1-nitro-2,6-dimethyl-4-*tert*-butyl benzene or 3,5-dinitro-2,6-dimethyl-4-*tert*-butyl benzene or 1,3,5-trinitro-2,6-dimethyl-4-*tert*-butyl benzene was weighed and to it 300ml sesame oil was added. The mixture was heated to achieve dissolution for later use. 100g chrysanthemum, 100g Japanese polygala, were weighed and steamed to give 500ml liquid. After filtration, the liquid was poured to the previous solution, mixed well, bottled and steam sterilized to afford oral liquid.

### Example 21 oral nutrient

300mg 1-nitro-2,6-dimethyl-4-*tert*-butyl benzene or 3,5-dinitro-2,6-dimethyl-4-*tert*-butyl benzene or 1,3,5-trinitro-2,6-dimethyl-4-*tert*-butyl benzene was weighed and to it 300ml sesame oil was added. The mixture was heated to achieve dissolution. Honey at a dilution of 1:10 was added up to 5000ml. It was then bottled and autoclaved.

### Example 22 injection

### A method for the preparation of nitrobenzene sodium injection.

500ml water for injection was mixed with 500mg powder of nitrobenzene sodium, followed by bottling and steam sterilization, to afford injection in 0.2mg/2ml and 0.3mg/3ml standards, for intramuscular and intravenous administration.

## Claims

1. A use of at least one compound of Formula I in the manufacture of pharmaceutical composition for the prophylaxis and treatment of diseases , injuries or symptoms caused by bacteria, fungi, 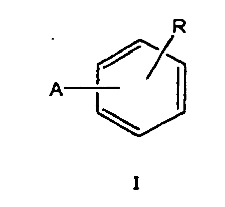 viruses,
wherein, A is one, two or three nitro groups, at 1, 3, or 5 position on the benzene ring; R is one, two or three linear or branched C₁₋₆ alkyl groups, at 2, 4 or 6 position on the benzene ring.

2. Use according to claim 1, wherein, in the compound of Formula I, A is only one nitro group at 1 position on the benzene ring, R is CH₃, C(CH₃)₃ and CH₃ at 2, 4 and 6 position respectively on the benzene ring.

3. Use according to claim 1, wherein, in the compound of Formula I, A is three nitro groups at 1,3, 5 positions respectively on the benzene ring, R is CH₃, C(CH₃)₃ and CH₃ at 2, 4 and 6 position respectively on the benzene ring.

4. Use according to claim 1, wherein, in the compound of Formula I, A is two nitro groups at 3,5 positions respectively on the benzene ring, R is CH₃, C(CH₃)₃ and CH₃ at 2, 4, and 6 position respectively on the benzene ring.

5. Use according to claim 1, wherein, in the compound of Formula i, A is one nitro group at 1 position on the benzene ring, R is C(CH₃)₃, CH₃, and CH₃ at 2, 4 and 6 position respectively on the benzene ring.

6. Use according to claim 1, wherein said compound of Formula I selected from:
1,3,5-trinitro-2,6-dimethyl-4-*tert*-butyl benzene;
1,3-dinitro-2-6-dirmethyl-4-*tert*-butyl benzene;
1,5-dinitro-2,6-dimethyl-4-*tert*-butyl benzene;
3,5-dinitro-2,6-dimethyl-4- *tert*-butyl benzene;
1-nitro-2,6-dimethyl-4-*tert*-butyl benzene;
3-nitro-2,6-dimethyl-4-*tert*-butyl benzene;
5-nitro-2,6-dimethyl-4-*tert*-butyl benzene;
1,3,5-tinitro-2,4,6-*tri-tert*-butyl benzene;
1,3-dinitro-2,4,6-*tri-tert*-butyl benzene;
3,5-dinitro-2,4,6-*tri-tert*-butyl benzene;
1,5-dinitro-2,4,6-*tri-tert*-butyl benzene;
1-nitro-2,4,*6-tri-tert-butyl* benzene;
3-nitro-2,4,6-*tri-tert*-butyl benzene;
5-nitro-2,4,6-*tri-tert*-butyl benzene;
1,3,5-trinitro-2,4,6-tri-methyl benzene;
1,3-dinitro-2,4,6-tri-methyl benzene;
3,5-dinitro-2,4,6-tri-methyl benzene;
1,5-dinitro-2,4,6-tri-methyl benzene;
1-nitro-2,4,6-tri-methyl benzene;
3-nirto-2,4,6-tri-methyl benzene;
5-nitro-2,4,6-tri-methyl benzene; and
1-nitro-2,6-dimethyl-4-*tert*-butyl benzene.

7. Use according to claim 1, wherein said diseases and/or symptoms are: virus hepatitis, tuberculosis, poliomyelitis, typhoid, bacillary dysentery, cholera, influenza, chickenpox, herpes zoster, parotitis, scarlet fever, diphtheria, pertussis, encephalitis B, viral encephalitis, malaria, visceral leishmaniasis, rabies, plague, anthrax, brucellosis, leptospirosis.

8. Use according to claim 1, wherein said diseases and/or symptoms are: osteomyelitis, encephalitis, optic neuritis, conjunctivitis, otitis media, paranasal sinusitis, stomatitis, pharyngitis, gingivitis, periodontitis, tonsillitis, bronchitis, pneumonia, gastroenteritis, cholecystitis, cystitis, pancreatitis, arteritis, phlebitis, neuritis, vaginitis, vulvitis, orchitis, salpingitis, ovaritis, endometritis, prostatitis, psoriasis.

9. Use according to claim 1, wherein said diseases and/or symptoms are: viral dermatosis, coccigenic dermatosis, bacillary dermatiosis, fungal dermatosis, spirochetal dermatosis, zoogenetic dermatosis, occupational dermatosis, neurotic dermatosis, allergodermia, physicogenic dermatosis, photogenous dermatosis, allergic dermatosis, lichen planus and lichenoid rash, erythemoid dermatosis, erythroderma, desquanmativum, vesicular dermatosis, keratinizing dermatosis, pigmentary dematosis, dermovascular disease and lymphodermia, nutritional and metabolic dermatiosis, disease of dermis and hypodermis, reticulosis, skin syndrom, granduloma annulare, pustulodermia, dermatitis continuee of extremities, edeomycodermatitis such as cervical erosion, neuodermatitis, ulcerative disease of gastrointestinal tract and mouth cavity.

10. Use according to claim 1, wherein said pharmaceutical compositions can be administered orally, parenterally or locally.

11. Use according to claim 1, wherein parenteral administration includes intramuscular, subcutaneous, intravenous, drip administration.

## Patentansprüche

1. Verwendung mindestens einer Verbindung mit der Formel I bei der Herstellung einer pharmazeutischen Zusammensetzung zur Prophylaxe und Therapie von Krankheiten, Verletzungen oder Symptomen, die durch Bakterien, Pilze, 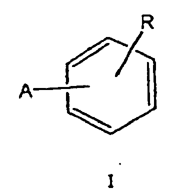 Viren hervorgerufen werden,
wobei es sich bei A um eine, zwei oder drei Nitrogruppen an der Stelle 1, 3 oder 5 am Benzolring handelt; es sich bei R um eine, zwei oder drei lineare oder verzweigte C₁₋₆ Alkylgruppen an der Stelle 2, 4 oder 6 am Benzolring handelt.

2. Verwendung nach Anspruch 1,
wobei es sich bei der Verbindung mit der Formel I bei A um nur eine Nitrogruppe an der Stelle 1 am Benzolring handelt, es sich bei R um CH₃, C(CH₃)₃ bzw. CH₃ an der Stelle 2, 4 und 6 am Benzolring handelt.

3. Verwendung nach Anspruch 1,
wobei es sich bei der Verbindung mit der Formel I bei A um jeweils drei Nitrogruppen an den Stellen 1, 3, 5 am Benzolring handelt, es sich bei R um CH₃, C(CH₃)₃ bzw. CH₃ an der Stelle 2, 4 und 6 am Benzolring handelt.

4. Verwendung nach Anspruch 1,
wobei es sich bei der Verbindung mit der Formel I bei A um jeweils zwei Nitrogruppen an den Stellen 3, 5 am Benzolring handelt, es sich bei R um CH₃, C(CH₃)₃ bzw. CH₃ an der Stelle 2, 4 und 6 am Benzolring handelt.

5. Verwendung nach Anspruch 1,
wobei es sich bei der Verbindung mit der Formel I bei A um eine Nitrogruppe an der Stelle 1 am Benzolring handelt, es sich bei R um C(CH₃)₃, CH₃ bzw. CH₃ an der Stelle 2, 4 und 6 am Benzolring handelt.

6. Verwendung nach Anspruch 1,
wobei die Verbindung mit der Formel I ausgewählt ist aus:
1, 3, 5-Trinitro-2,6-dimethyl-4-*tert*-butylbenzol;
1,3-Dinitro-2-6-dimethyl-4-*tert*-butylbenzol;
1,5-Dinitro-2,6-dimethyl-4-*tert*-butylbenzol;
3,5-Dinitro-2,6-dimethyl-4-*tert*-butylbenzol;
1-Nitro-2,6-dimethyl-4-*tert*-butylbenzol;
3-Nitro-2,6-dimethyl-4-*tert*-butylbenzol;
5-Nitro-2,6-dimethyl-4-*tert*-butylbenzol;
1,3,5-Trinitro-2,4,6-*tri-tert*-butylbenzol;
1,3-Dinitro-2,4,6-*tri-tert*-butylbenzol;
3,5-Dinitro-2,4,6-*tri*-*tert*-butylbenzol;
1,5-Dinitro-2,4,6-*tri-tert*-butylbenzol;
1-Nitro-2,4,6-*tri-tert*-butylbenzol;
3-Nitro-2,4,*6-tri-tert-* butylbenzol;
5-Nitro-2,4,6-*tri-tert*-butylbenzol;
1,3,5-trinitro-2,4,6-trimethylbenzol;
1,3-Dinitro-2,4,6-trimethylbenzol;
3,5-Dinitro-2,4,6-trimethylbenzol;
1,5-Dinitro-2,4,6-trimethylbenzol;
1-Nitro-2,4,6-trimethylbenzol;
3-Nitro-2,4,6-trimethylbenzol;
5-Nitro-2,4,6-trimethylbenzol; und
1-Nitro-2,6-dimethyl-4-*tert*-butylbenzol.

7. Verwendung nach Anspruch 1,
wobei es sich bei den Krankheiten und/oder Symptomen handelt um:
Virushepatitis, Tuberkulose, Poliomyelitis, Typhus, bakterielle Ruhr, Cholera, Influenza, Windpocken, Herpes Zoster, Parotitis, Scharlach, Diphterie, Keuchhusten, Enzephalitis B, virale Enzephalitis, Malaria, viszerale Leishmaniase, Tollwut, Pest, Anthrax, Brucellose, Leptospirose.

8. Verwendung nach Anspruch 1,
wobei es sich bei den Krankheiten und/oder Symptomen handelt um:
Osteomyelitis, Enzephalitis, Augennervneuritis, Konjunktivitis, Otitis media, paranasale Sinusitis, Stomatitis, Pharyngitis, Gingivitis, Periodontitis, Tonsillitis, Bronchitis, Pneumonie, Gastroenteritis, Cholezystitis, Zystitis, Pankreatitis, Arteritis, Phlebitis, Neuritis, Vaginitis, Vulvitis, Orchitis, Salpingitis, Ovaritis, Endometritis, Prostatitis, Psoriasis.

9. Verwendung nach Anspruch 1,
wobei es sich bei den Krankheiten und/oder Symptomen handelt um:
Virusdermatose, Kokzidiendermatose, Bakteriendermatose, Pilzdermatose, Spirochätendermatose, Dermatozoonose, berufsbedingte Dermatose, neurotische Dermatose, Allergodermie, physisch bedingte Dermatose, Lichtdermatose, allergische Dermatose, Lichen planus und lichenoides Exanthem, Erythemdermatose, Erythrodermie, Desquamation, Vesikulärdermatose, Keratose, Pigmentdermatose, dermovaskuläre Erkrankung und Lymphodermie, ernährungs- und stoffwechselbedingte Dermatose, Erkrankung der Dermis und Hypodermis, Retikulose, Skin-Syndrom, Granuloma annulare, Pustulose, anhaltende Dermatitis der Extremitäten, Endeomycodermatitis wie Zervix-Erosion, Neurodermitis, ulzeröse Erkrankung das Magen-/Darmtrakts und der Mundhöhle.

10. Verwendung nach Anspruch 1,
wobei die pharmazeutischen Zusammensetzungen oral, parenteral oder lokal verabreicht werden können.

11. Verwendung nach Anspruch 1,
wobei die parenterale Verabreichung intramuskuläre, subkutane, intravenöse Tropfverabreichung umfasst.

## Revendications

1. Utilisation d'au moins un composé de Formule I dans la fabrication d'une composition pharmaceutique pour la prophylaxie et le traitement de maladies, de blessures ou de symptômes causés par des bactéries, des champignons 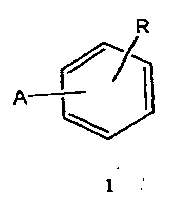 des virus,
dans laquelle A est un, deux ou trois groupes nitro en position 1, 3 ou 5 sur l'anneau benzène ; R est un, deux ou trois groupes alkyle C₁₋₆ linéaire ou multibrin en position 2, 4 ou 6 sur l'anneau benzène.

2. Utilisation selon la revendication 1, dans laquelle, dans le composé de Formule I, A est seulement un groupe nitro en position 1 sur l'anneau benzène ; R est CH₃, C(CH₃)₃ et CH₃ respectivement en position 2, 4 et 6 sur l'anneau benzène.

3. Utilisation selon la revendication 1, dans laquelle, dans le composé de Formule I, A est trois groupes nitro respectivement en position 1, 3, 5 sur l'anneau benzène ; R est CH₃, C(CH₃)₃ et CH₃ respectivement en position 2, 4 et 6 sur l'anneau benzène.

4. Utilisation selon la revendication 1, dans laquelle, dans le composé de Formule I, A est deux groupes nitro respectivement en position 3, 5 sur l'anneau benzène ; R est CH₃, C(CH₃)₃ et CH₃ respectivement en position 2, 4 et 6 sur l'anneau benzène.

5. Utilisation selon la revendication 1, dans laquelle, dans le composé de Formule I, A est un groupe nitro en position 1 sur l'anneau benzène ; R est C(CH₃)₃, CH₃ et CH₃ respectivement en position 2, 4 et 6 sur l'anneau benzène.

6. Utilisation selon la revendication 1, dans laquelle le composé de Formule I est choisi parmi :
1, 3, 5-trinitro-2,6-diméthyl-4-*tert*-butyle benzène;
1,3-dinitro-2-6-diméthyl-4-*tert*-butyle benzène;
1,5-dinitro-2,6-diméthyl-4-*tert-*butyle benzène;
3,5-dinitro-2,6-diméthyl-4-*tert*-butyle benzène;
1-nitro-2,6-diméthyl-4-*tert*-butyle benzène;
3-nitro-2,6-diméthyl-4-*tert*-butyle benzène;
5-nitro-2,6-diméthyl-4-*tert*-butyle benzène;
1,3,5-trinitro-2,4,6-*tri-tert*-butyle benzène;
1,3-dinitro-2,4,6-*tri-tert*-butyle benzène;
3,5-dinitro-2,4,6-*tri-tert*-butyle benzène;
1,5-dinitro-2,4,6-*tri-tert*-butyle benzène;
*1-nitro-2,4,6-tri-tert-butyle* benzène;
3-nitro-2,4,6-*tri-tert*-butyle benzène;
5-nitro-2,4,6-*tri-tert-*butyle benzène;
1,3,5-trinitro-2,4,6-triméthyle benzène;
1,3-dinitro-2,4,6-triméthyle benzène;
3,5-dinitro-2,4,6-triméthyle benzène;
1,5-dinitro-2,4,6-triméthyle benzène;
1-nitro-2,4,6-triméthyle benzène;
3-nitro-2,4,6-triméthyle benzène;
5-nitro-2,4,6-triméthyle benzène; et
1-nitro-2,6-diméthyl-4-*tert*-butyle benzène.

7. Utilisation selon la revendication 1, dans laquelle, pour lesdits symptômes et maladies, il s'agit de : hépatite virale, tuberculose, poliomélythe, tiphoïde, dysenterie bacillaire, choléra, influenza, varicelle, herpès zoster, parotite, scarlatine, diphtérie, coqueluche, encéphalite B, encéphalite virale, malaria, leishmaniase viscérale, rage, peste, anthrax, brucellose, leptospirose.

8. Utilisation selon la revendication 1, dans laquelle, pour lesdits symptômes et maladies, il s'agit de : ostéomyélite, encéphalite, névrite oculaire, conjonctivite, otite moyenne, sinusite paranasale, stomatite, pharyngite, gingivite, périodontite, amygdalite, bronchite, pneumonie, gastroentérite, cholécystite, cystite, pancréatite, artérite, phlébite, névrite, vaginite, vulvite, orchite, salpingite, ovarite, endométrite, prostatite, psoriasis.

9. Utilisation selon la revendication 1, dans laquelle, pour lesdits symptômes et maladies, il s'agit de : dermatose virale, dermatose coccigène, dermatose bactérienne, dermatose fongique, dermatose spirochétale, dermatozoonose, dermatose professionnelle, dermatose névrosée, allergodermie, dermatose d'origine physique, dermatose photogène, dermatose allergique, lichen plan et éruption lichénoide, dermatose érythème, érythrodermie, desquamation, dermatose vésiculaire, dermatose kératisée, dermatose pigmentaire, maladie dermovasculaire et lymphodermie, dermatose nutritionnelle et métabolique, maladie du derme et de l'épiderme, réticulose, syndrome de la peau, granulome annulaire, pustulose, dermatite continue des extrémités, endeomycodermatite telle l'érosion cervicale, neurodermatite, les maladies à ulcère du tractus gastrointestinal et de la cavité bucale.

10. Utilisation selon la revendication 1, dans laquelle, les préparations pharmaceutiques peuvent être administrées oralement, parentéralement ou localement.

11. Utilisation selon la revendication 1, dans laquelle l'administration parentérale comprend l'administration en gouttes intramusculaire, subcutanée, intraveineuse.
